(19) Europäisches Patentamt
European Patent Office
Office européen des brevets

(11) Veröffentlichungsnummer: **0 212 301 B1**

(12) # EUROPÄISCHE PATENTSCHRIFT

(45) Veröffentlichungstag der Patentschrift: **24.10.90**

(51) Int. Cl.$^5$: **C 07 C 319/14,** C 07 C 323/18

(21) Anmeldenummer: **86110101.2**

(22) Anmeldetag: **23.07.86**

(54) **Verfahren zur Herstellung von 2-Aminophenyl-thioethern.**

(30) Priorität: **05.08.85 DE 3528033**

(43) Veröffentlichungstag der Anmeldung:
**04.03.87 Patentblatt 87/10**

(45) Bekanntmachung des Hinweises auf die Patenterteilung:
**24.10.90 Patentblatt 90/43**

(84) Benannte Vertragsstaaten:
**CH DE FR GB IT LI**

(56) Entgegenhaltungen:
**EP-A-0 039 483**
**FR-A- 657 183**
**US-A-2 007 335**
**US-A-3 102 142**

(73) Patentinhaber: **HOECHST AKTIENGESELLSCHAFT**
**Postfach 80 03 20**
**D-6230 Frankfurt am Main 80 (DE)**

(72) Erfinder: **Papenfuhs, Theodor, Dr.**
**Heinrich-Bleicher-Strasse 40**
**D-6000 Frankfurt am Main 50 (DE)**

EP 0 212 301 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der vorliegenden Anmeldung ist ein hinsichtlich Gewerbehygiene und Abwasserentsorgung sowie hinsichtlich der Ausbeute verbessertes Verfahren zur Herstellung von 2-Aminophenyl-thioethern, welche wertvolle Zwischenprodukte zur Herstellung von Farbstoffen, Pflanzenschutzmitteln und Pharmazeutika sind.

2-Aminophenyl-thioether der allgemeinen Formel (1)

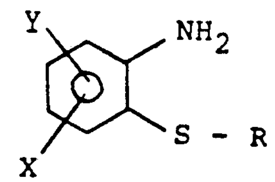

(1)

in welcher R eine $Alkyl_{C_1-C_6}$gruppe, die durch Hydroxyl-, $Alkoxy_{C_1-C_4}$, Carboxyl-, —COO-$Alkyl_{C_1-C_4}$, $Alkenyl_{C_2-C_6}$ oder Phenylgruppen oder den Rest

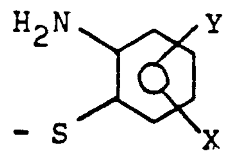

substituiert sein kann, und X und Y je ein Wasserstoff- oder Halogenatom, beispielsweise in Chlor- oder Bromatom, oder eine $Alkyl_{C_1-C_6}$, $Alkoxy_{C_1-C_6}$ oder Nitrogruppe darstellen, konnten bisher technisch durch Alkylierung entsprechender 2-Nitrophenyl-mercaptane mit Verbindungen der allgemeinen Formel (2)

$$R—Z \qquad (2)$$

in welcher R die vorstehend genannte Bedeutung hat und Z ein Halogenatom, beispielsweise ein Chlor- oder Bromatom darstellt, oder mit Verbindungen der allgemeinen Formel (2a)

$$(R'O_2SO_2 \qquad (2a)$$

in welcher R' eine $Alkyl_{C_1-C_4}$ gruppe bedeutet, oder mit Verbindungen der allgemeinen Formel (2b)

$$Z\text{-}alkylen_{C_2-C_6}\,Z \qquad (2b)$$

in welcher Z die vorstehend genannte Bedeutung hat, oder mit einem $Alkylen_{C_2-C_3}$oxid und anschließende Reduktion der Nitrogruppe, oder durch entsprechende Alkylierung der zugrundeliegenden 2-Aminophenylmercaptane, die durch Reduktion der vorstehend genannten Nitrovorstufen, in Einzelfällen auch durch alkalische Totalhydrolyse von 2-Aminobenzthiazolen oder Benzthiazthioniumhalogeniden erhalten werden.

Diese Synthesevarianten verlaufen somit über freie Arylmercaptane, wodurch sich erhebliche Probleme hinsichtlich Gewerbehygiene und Abwasserentsorgung ergeben. Außerdem bestand auch aus chemischer Sicht aus folgenden Gründen ein Bedürfnis nach einem effektivieren Verfahren zur technisch praktikablen Herstellung der Zielverbindungen:

a) Die Ausgangsprodukte der ersten Synthesevariante, die 2-Nitrophenyl-mercaptane, sind sehr oxidationsempfindlich und machen durch Übergang in 2-Nitrophenyldisulfide Ausbeuteminderungen unvermeidbar.

b) Die Alkylierung der 2-Aminophenylmercaptane ist in der Regel wenig selektiv, sodaß eine aufwendige Abtrennung von N-Alkylaminophenyl-mercaptanen bzw. -thioethern vom Zielprodukt unabdingbar ist, wodurch ebenfalls Ausbeuteverluste in Kauf genommen werden müssen.

Es wurde nun überraschenderweise gefunden, daß man die 2-Aminophenyl-thioether der vorstehend genannten allgemeinen Formel (1) unter vollständiger Vermeidung der erwähnten Nachteile der bekannten Verfahren herstellen kann, indem man die in bekannter Weise EP—PS 0 039 483 durch Umsetzung von 2-Aminobenzthiazolen der allgemeinen Formel (3)

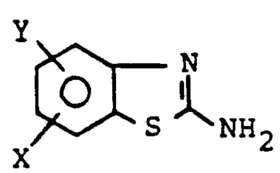

(3)

in welcher X und Y die vorstehend genannten Bedeutungen haben, mit Alkalimetall- oder Erdalkalimetallhydroxiden in einem alkalibeständigen wasserfreien oder praktisch wasserfreien Lösungsmittel, wie Ethanol, Isobutanol, 1,2-Dihydroxypropan oder 1,3-Dihydroxypropan, vorzugsweise Ethylenglykol, Glycerin, Ethylenglykol-monomethylether oder Ethylenglykol-monoethylether, als Zwischenverbindungen in hohen Ausbeuten erhältlichen Salze der allgemeinen Formel (4)

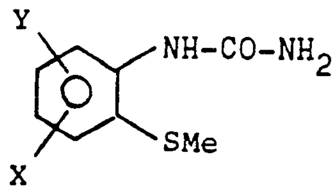

(4)

in welcher X und Y die vorstehend genannten Bedeutungen haben und Me ein Alkalimetallatom oder die äquivalente Menge eines Erdalkalimetallatoms, vorzugsweise ein Natrium-, Kalium,- 1/2-Calzium- oder 1/2-Magnesiumatom, bedeutet, in isolierter Form oder vorteilhaft in der im Ringöffnungsgemisch vorliegenden Suspension, mit einem Alkylierungsmittel der allgemeinen Formel (2)

$$R—Z \qquad (2)$$

in welcher R die vorstehend genannte Bedeutung hat und Z ein Halogenatom, beispielsweise ein Chlor- oder Brom-atom darstellt, oder der allgemeinen Formel (2a)

$$(R'O)_2SO_2 \qquad (2a)$$

in welcher R' eine $Alkyl_{\overline{C_1-C_4}}$gruppe bedeutet, oder der allgemeinen Formel (2b)

$$Z\text{-}alkylen_{\overline{C_2-C_6}}\,Z \qquad (2b)$$

in welcher Z die vorstehend genannte Bedeutung hat, oder mit einem $Alkylen_{\overline{C_2-C_3}}$ oxid bei Temperaturen von 0°C bis 100°C, vorzugsweise 20° bis 70°C, umsetzt.

Die hierbei intermediär gebildeten 2-Alkylmercaptophenylharnstoffe der allgemeinen Formel (5)

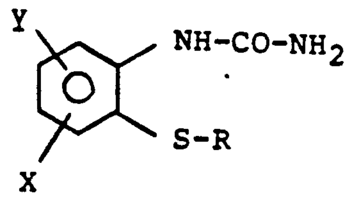

(5)

in welcher R, X und Y die weiter oben genannten Bedeutungen haben, sind nur in Ausnahmefällen genügend hydrolysestabil und können lediglich bei absolut wasserfreiem Arbeiten isoliert werden. In der Regel werden sie unter den Reaktionsbedingungen vom im Reaktionsgemisch enthaltenen oder nachfolgend zugesetzten Wasser in kurzer Zeit zu den Endprodukten der genannten allgemeinen Formel (1) unter Bildung von Kohlendioxid und Ammoniak gemäß dem Reaktionsschema

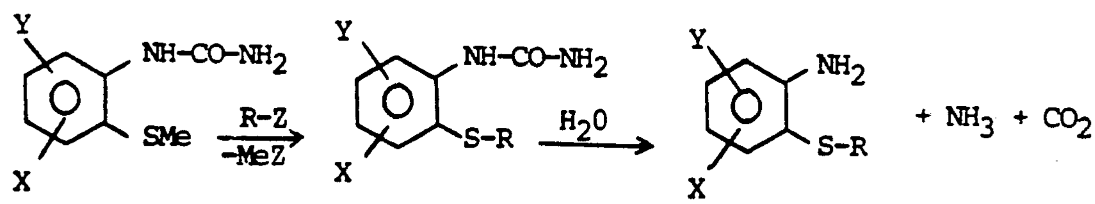


gespalten.

Somit gelingt es nach dem erfindungsgemäßen Verfahren, die technisch leicht zugänglichen 2-Aminobenzthiazole DE—PS—2 801 991 in einer Eintopfreaktion durch Umsetzung mit Alkalimetall- oder Erdalkalimetallhydroxiden und nachfolgende Behandlung mit einem Alkylierungsmittel der genannten Formeln (2), (2a), (2b) oder mit einem Alkylenoxid in hoher Ausbeute und Reinheit in 2-Aminophenylthioether zu überführen. Das erfindungsgemäße Verfahren vermeidet den Einsatz freier Mercaptane und bietet daher keinerlei ökologische oder gewerbehygienische Probleme. Dadurch und verbunden mit der erzielbaren höheren Ausbeute stellt das erfindungsgemäße Verfahren auch einen erheblichen technischen Fortschritt dar.

Es ist zudem überraschend, daß beim erfindungsgemäßen Verfahren keine merkliche Bildung von Nebenprodukten beobachtet wird. Zwar ist das Fehlen jeglicher N-Alkylierung infolge des durch die Carbamoylgruppe bewirkten Schutzes der Aminogruppe erklärbar, doch war zu erwarten, daß beim Alkylierungsschritt (in Gegenwart von Wasser), wenn auch nur in Spuren und örtlich begrenzt freiwerdende Säure den sehr leicht ablaufenden, sauer katalysierten Ringschluß des 2-Mercaptophenylharnstoffs zum entsprechenden 2-Hydroxybenzthiazol gemäß EP—PS—0 039 483 bzw. die ebenfalls sauer katalysierte Hydrolyse der Harnstoffgruppierung zum 2-Aminothiophenol ermöglicht und dadurch durch 2-Hydroxybenzthiazol und/oder 2-(Alkyl)aminothiophenol-(ether) verunreinigte 2-Aminophenyl-thioether resultieren.

Daß diese erwarteten Nebenreaktionen beim erfindungsgemäßen Verfahren allenfalls in Spuren ablaufen, somit eine praktisch einheitliche Reaktion zu den gewünschten Zielprodukten der Formel (1) resultiert, war nicht vorauszusehen und erscheint auf Grund des komplexen Reaktionsgeschehens in mehreren Syntheseschritten äußerst überraschend.

Das erfindungsgemäße Verfahren wird im einzelnen in der Weise durchgeführt, daß man das beispielsweise gemäß EP—PS—0 039 483, Beispiel 1, Absatz 1 erhältliche 2-Mercaptophenylharnstoffsalz der genannten allgemeinen Formel (4) in Form der beim Verdünnen des Ringöffnungsansatzes mit Wasser resultierenden wäßrigen Lösung (oder ggf. Suspension) bei Temperaturen von 0—100°C, vorzugsweise 20—70°C, mit einem Alkylierungsmittel der genannten allgemeinen Formeln (2), (2a) oder (2b), beispielsweise einem Dialkylsulfat, einem Alkylendihalogenid oder einem Alkylhalogenid, oder mit einem Alkylenoxid, z.B. Ethylen- oder Propylenoxid, in mindest molarer Menge, vorzugsweise in einem Überschuß von 5—25%, innerhalb von 0 bis 5, vorzugsweise 0,5 bis 2 Stunden versetzt, den gebildeten 2-Aminophenyl-thioether durch Phasentrennung, Filtration oder Extraktion vom Reaktionsgemisch abtrennt und nachfolgend vom anhaftenden Wasser oder Extraktionsmittel befreit.

Selbstverständlich kann man auch von gemäß EP—PS—0 039 483, Beispiel 1, (Absatz 1) herstellbarem isoliertem freiem 2-Mercaptophenylharnstoff ausgehen, diesen nach Suspendieren in Wasser mit molarer Menge Alkalimetall- oder Erdalkalimetallhydroxid oder -carbonat zum Salz der genannten allgemeinen Formel (4) neutralisieren und von da aus in das erfindungsgemäße Verfahren eintreten. Wegen der eingangs geschilderten Problematik bei der Verwendung freier Mercaptane stellt diese Variante jedoch keine bevorzugte dar.

Besonders bevorzugt ist dagegen die nachfolgende Variante, bei welcher die aus dem Salz des 2-Mercaptophenylharnstoffes der genannten Formel (4) und dem zur Ringöffnung verwandten Lösungsmittel (Alkanol, Glykol, Glycerin, Glykolmonoether) bestehende Reaktionsmischung, oder ein aus einem isolierten 2-Mercaptophenylharnstoff-Salz der genannten Formel (4) [EP—PS—0 039 483, Beispiele 6—10] und einem der zur Ringöffnung verwandten, oder einem anderen unter Reaktionsbedingungen gegenüber dem Alkylierungsmittel (Formeln 2)) oder dem Alkylenoxid inerten Lösungsmittel (Toluol, Xylole, Chlorbenzol, Chlortoluole, Benzin, Chloraliphaten) hergestelltes Ausgangsgemisch mit dem Alkylierungsmittel (Formeln 2)) oder dem Alkylenoxid bei Temperaturen von 0 bis 200°C, vorzugsweise 20 bis 120°C, in mindestens molarer Menge, vorzugsweise in einem Überschuß von 5 bis 50%, innerhalb von 1 bis 10, vorzugsweise 2 bis 6 Stunden, gegebenenfalls unter Druck, umgesetzt wird. Zur Aufarbeitung wird das Zielprodukt (Formel 1) durch Filtration, Auswaschen des Lösungsmittels und des gebildeten Salzes mit Wasser und Trocknen isoliert, oder, insbesondere bei flüssigen oder niedrig-schmelzenden oder im Lösungsmittel zu gut löslichen Zielprodukten, ggf. nach Filtration vom gebildeten Salz, destillativ konzentriert und dann durch Filtration, Wäsche und Trocknung oder besonders vorteilhaft durch Fraktionierung, vorzugsweise im Vakuum von 0,1 bis 100 Torr, in reiner Form gewonnen.

Die nachfolgenden Beispiele sollen das erfindungsgemäße Verfahren näher erläutern, ohne es zu beschränken. Die angegebenen Teile sind Gewichtsteile.

Beispiel 1

Die gemäß Beispiel 3 der EP—PS—0 039 483 beim Umsatz von 82 Teilen 4-Methyl-2-aminobenzthiazol mit 50 Teilen Ätznatron in Glycerin erhaltene, mit Kohle geklärte Lösung des Natriumsalzes des 6-Methyl-2-mercapto-phenylharnstoffes wird innerhalb von 15 Minuten unter Rühren bei 20 bis 25°C mit 82 Teilen Dimethylsulfat versetzt. Anschließend läßt man 45 Minuten nachregieren.

Man trennt die gebildete organische Unterphase (Dichte 1,15 g/ml) und fraktioniert sie über eine kurze Kolonne. Nach einem geringen Vorlauf (Wasser) gehen bei 20 Torr/132°C 72 Teile 3-Methylmercapto-2-aminotoluol der Formel

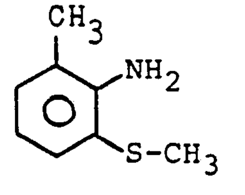

über, was einer Ausbeute von 94,1% der Theorie, bezogen auf eingesetztes 4-Methyl-2-aminobenzthiazol, entspricht.

Das Produkt ist praktisch frei von Verunreinigungen, zeigt im Gaschromatogramm einen Reingehalt >99%, der sich auch durch den Diazotierwert von 99,5% bestätigt.

Beispiel 2

168 Teile des gemäß Beispiel 1, Absatz 1 der EP—PS—0 039 483 zugänglichen 2-Mercaptophenylharnstoffes werden in 500 Teilen Wasser suspendiert, mit 60 Teilen Kaliumhydroxid versetzt und die erhaltene Mischung in einen 1 1-Autoklav überführt. Man verschließt den Autoklav, drückt 75 Teile Methylchlorid auf und heizt in 1 Stunde auf 80°C, wobei sich ein Innendruck von ca. 2 bar einstellt, der sich beim 1-stündigen Nachrühren bei 80°C fast vollständig abbaut. Nach Abkühlen wird der Autoklav entleert, die gebildete organische Unterphase (Dichte 1,14 g/ml) abgetrennt und über eine kurze Kolonne fraktioniert. Als Hauptlauf gehen bei 15 Torr/134°C 130 Teile 2-Methylmercaptoanilin der Formel

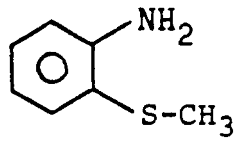

über, was einer Ausbeute von 93,5% der Theorie, bezogen auf eingesetzten 2-Mercaptophenyl-harnstoff, entspricht.

Das Produkt hat einen Diazotierwert von 99,6% und erweist sich im Geschromatogramm als einheitlich.

Beispiele 3—8

Arbeitet man nach den Angaben des Beispiels 2, ersetzt aber das Methylchlorid durch aliquote Teile der in der nachstehenden Tabelle 1 angegebenen Alkylierungsmittel, so erhält man ebenfalls erfindungsgemäß 2-Aminophenyl-alkyl-ether der Formel (6)

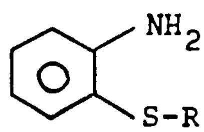

(6)

mit den in der Tabelle 1 aufgeführten Siedepunkten und Ausbeuten:

Tabelle 1

| Beispiel | Alkylierungsmittel | Zielprodukt (R=) | Siedepunkt | Ausbeute |
|---|---|---|---|---|
| 3 | $(C_2H_5O)_2SO_2$ | $-C_2H_5$ | 144°C/15Torr | 92,9 % |
| 4 | 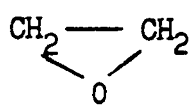 | $-CH_2CH_2-OH$ | 191°C/15Torr | 89,8 % |
| 5 | $n-C_3H_7Cl$ | $-(CH_2)_2-CH_3$ | 98°C/1 Torr | 93,8 % |
| 6 | $CH_2=CH-CH_2Br$ | $-CH_2-CH=CH_2$ | 110°C/1 Torr | 89,5 % |
| 7 | 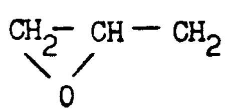 | $-CH_2-CHOH-CH_3$ | 142°C/1 Torr | 87,0 % |
| 8 | $n-C_4H_9Cl$ | $-(CH_2)_3-CH_3$ | 105°C/1 Torr | 94,8 % |

Beispiel 9

112, 25 Teile des gemäß Beispiel 6 der EP—PS—0 039 483 erhältlichen Natriumsalzes des 4-Chlor-2-mercaptophenylharnstoffes werden in 500 Teilen Wasser von Raumtemperatur suspendiert. Anschließend

tropft man unter Rühren innerhalb von 30 Minuten 100 Teile Diethylsulfat zu, wobei die Innentemperatur 25°C nicht übersteigen soll. Dann heizt man in 45 Minuten auf 80°C, rührt 30 Minuten nach, trennt die gebildete organische Unterphase ab und fraktioniert sie im Vakuum. Nach einem unbedeutenden wäßrigen Vorlauf gehen bei 20 Torr/167°C 90,0 Teile 2-Ethyl-mercapto-4-chloranilin der Formel

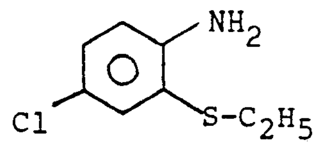

über, was einer Ausbeute von 96,0% der Theorie, bezogen auf eingesetztes Natriumsalz des 4-Chlor-2-mercaptophenylharnstoffes, entspricht.

Das Produkt ist chromatografisch einheitlich und hat einen Diazotierwert von 99,7%.

Beispiele 10—15

Ersetzt man in Beispiel 9 das Natriumsalz des 4-Chlor-2-mercaptophenyl-harnstoffes durch aliquote Teile der in der nachstehenden Tabelle 2 aufgeführten Salze von 2-Mercaptophenylharnstoffen der Formel (7)

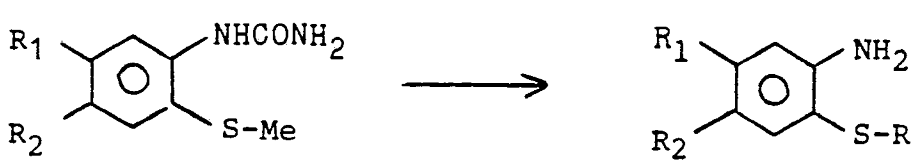

(7) (8)

und das Diethylsulfat durch aliquote Teile des ebenfalls in Tabelle 2 aufgeführten Alkylierungsmittels, so erhält man die 2-Alkylmercaptoaniline der Formel (8) mit den angegebenen Schmelz- bzw. Siedepunkten in den aus der Tabelle ersichtlichen Ausbeuten:

Tabelle 2

| Beispiel | Edukt (7) | | | Alkylierungsmittel | Produkt (8) | | | $Kp_{(Torr.)}/Fp.$ | Ausbeute |
|---|---|---|---|---|---|---|---|---|---|
| | Me | $R_1$ | $R_2$ | | R | $R_1$ | $R_2$ | | |
| 10 | Na | H | H | $Br-CH_2-CH_2-Br$ | $Br-CH_2-CH_2-Br$ | H | H | Fp. 78°C | 95,1 % |
| 11 | K | $CH_3$ | H | $(CH_3O)_2SO_2$ | $-CH_3$ | $CH_3$ | H | $Kp._{20}$:130°C | 91,5 % |
| 12 | K | H | $OCH_3$ | $(CH_3O)_2SO_2$ | $-CH_3$ | H | $OCH_3$ | $Kp._{20}$:142°C | 89,4 % |
| 13 | Mg/2 | Cl | H | $(C_2H_5O)_2SO_2$ | $-C_2H_5$ | Cl | H | $Kp._{20}$:164°C | 93,8 % |
| 14 | Na | Br | H | $(C_2H_5O)_2SO_2$ | $-C_2H_5$ | Br | H | $Kp._{20}$:176°C | 94,0 % |
| 15 | Ca/2 | Cl | H | 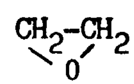 | $-CH_2CH_2OH$ | Cl | H | $Kp._5$:186°C | 87,8 % |

Beispiel 16

Eine Mischung aus 150 Teilen 2-Aminobenzthiazol, 150 Teilen festem Natriumhydroxid und 300 Teilen Ethylenglykol wird 6 Stunden bei 130—140°C gerührt (Beispiel 1 der EP—PS—0 039 483), dann auf 70°C abgekühlt und bei dieser Temperatur in 30 Minuten gleichmäßig mit 158 Teilen Benzylchlorid versetzt. Man rührt 2 Stunden bei 70 bis 80°C nach, läßt die Reaktionsmischung unter Rühren in 3000 Teile Wasser zügig einlaufen und kühlt auf 0°C ab. Das dabei ausgranulierte 2-Benzylmercaptoanilin der Formel

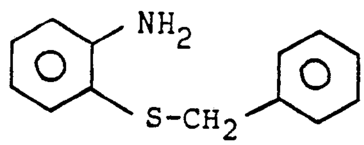

wird abgesaugt, mit Wasser gewaschen und im Vakuum bei Raumtemperatur getrocknet. Man erhält 190 Teile Produkt vom Schmelzpunkt 44°C, was einer Ausbeute von 88,4% der Theorie, bezogen auf eingesetztes 2-Amino-benzthiazol, entspricht.

Das Produkt ist chromatographisch einheitlich und zeigt einen Diazotierwert von 99,3%.

Beispiele 17—21

Ersetzt man in Beispiel 16 das 2-Aminobenzthiazol durch aliquote Teile der in der nachstehenden Tabelle 3 aufgeführten 2-Aminobenzthiazole der Formel (9)

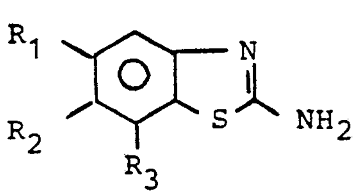

(9)

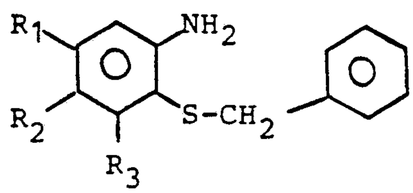

(10)

und arbeitet im übrigen in der angegebenen Weise, so erhält man die in der nachstehenden Tabelle 3 aufgeführten 2-Benzylmercaptoaniline der Formel (10) mit den dort angegebenen Schmelzpunkten und Ausbeuten.

Tabelle 3

| Beispiel | Edukt (9) | | | Produkt (10) | | | Schmelzpunkt | Ausbeute |
|---|---|---|---|---|---|---|---|---|
| | $R_1$ | $R_2$ | $R_3$ | $R_1$ | $R_2$ | $R_3$ | | |
| 17 | Cl | H | H | Cl | H | H | 54°C | 92,8 % |
| 18 | H | H | Cl | H | H | Cl | 41°C | 89,2 % |
| 19 | Cl | Cl | H | Cl | Cl | H | 72°C | 94,0 % |
| 20 | Br | H | H | Br | H | H | 56°C | 92,1 % |
| 21 | H | Cl | H | H | Cl | H | 61°C | 90,8 % |

## Beispiel 22

168 Teile des gemäß Beispiel 1, Absatz 1 der EP—PS—0 039 483 zugänglichen 2-Mercaptophenylharnstoffes werden in 500 Volumenteilen 1 n Natronlauge bei Raumtemperatur gelöst, auf einmal mit 195 Teilen β-Brompropionsäure versetzt und 2 Stunden bei Raumtemperatur verrührt. Dann heizt man innerhalb 1 Stunde auf 100°C, rührt 30 Minuten bei dieser Temperatur nach, kühlt auf 20°C, stellt mit verdünnter Salzsäure auf pH 3,0 und filtriert die ausgefallene 2-Aminophenyl-β-thiopropionsäure der Formel

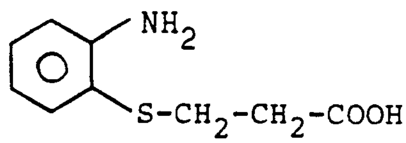

ab, wäscht mit Kaltwasser neutral und trocknet bei 60°C im Vakuum. Man erhält 162,5 Teile Produkt vom Schmelzpunkt 86 bis 87°C, was einer Ausbeute von 82,5% der Theorie, bezogen auf eingesetzten 2-Mercaptophenylharnstoff, entspricht. Das Produkt it chromatographisch einheitlich und zeigt einen Diazotierwert von 99,8%.

## Beispiel 23

Eine Mischung aus 150 Teilen 2-Aminobenzthiazol, 150 Teilen festem Natriumhydroxid und 300 Teilen Ethylenglykol wird 6 Stunden 130—140°C gerührt (Beispiel 1 der EP—PS—0 039 483). Man kühlt auf 40°C ab, versetzt dann so mit 113 Teilen Chloressigsäure, daß die Innentemperatur 50°C nicht übersteigt und rührt anschließend 2 Stunden bei dieser Temperatur nach. Dann heizt man auf 80 bis 90°C, hält 1 Stunde bei dieser Temperatur und läßt nachfolgend die gebildete Suspension des Natriumsalzes der 2-Aminophenylthioglykolsäure der Formel (11) in eine Mischung aus 2500 Teilen Wasser und 550 Teilen 30%iger Salzsäure einlaufen, wobei die Temperatur bis auf 60°C ansteigt. Bei dieser Prozedur cyclisiert die freigesetzte 2-Aminophenylthioglykolsäure rasch zum 3-Oxo-dihydrobenzthiazin der Formel (12), welche das stabile Anhydrid der instabilen freien Säure darstellt:

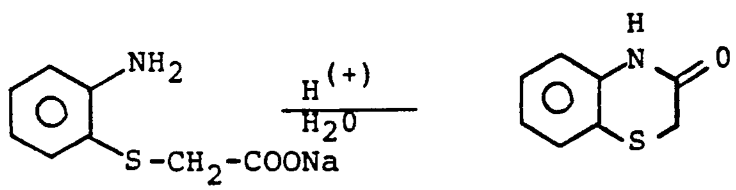


(11) (12)

Man rührt 30 Minuten bei 60 bis 70°C nach, kühlt auf Raumtemperatur ab, filtriert das ausgefallene Produkt ab, wäscht mit Wasser neutral und trocknet bei 120°C im Umluftschrank.

Man erhält 150 Teile 3-Oxo-dihydrobenzthiazin vom Schmelzpunkt 175°C, was einer Ausbeute von 90,0% der Theorie, bezogen auf eingesetztes 2-Aminobenzthiazol, entspricht.

Das Produkt ist chromatographisch rein und läßt sich durch Erwärmen mit Natronlauge zu einer klaren wäßrigen Lösung des 2-Aminophenylthioglykolsäure-Natriumsalzes öffnen, das, durch indirekte Diazotierung bestimmt, einen Reingehalt von 99,4% aufweist.

## Beispiele 24—30

Ersetzt man in Beispiel 23 das 2-Aminobenzthiazol durch aliquote Mengen seiner in der nachstehenden Tabelle 4 aufgeführten Derivate der Formel (13) und arbeitet im übrigen in der angegebenen Weise, so erhält man die mit Ausbeute und Schmelzpunkt in der Tabelle angegebenen 3-Oxo-dihydrobenzthiazine (14) in vergleichbarer Reinheit:

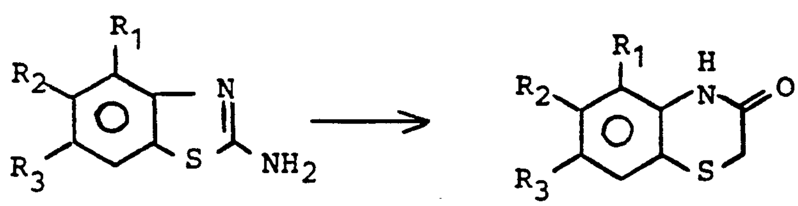

(13) (14)

Tabelle 4

| Beispiel | Edukt (13) | | | Produkt (14) | | | Schmelzpunkt | Ausbeute |
|---|---|---|---|---|---|---|---|---|
| | $R_1$ | $R_2$ | $R_3$ | $R_1$ | $R_2$ | $R_3$ | | |
| 24 | Cl | H | H | Cl | H | H | 153°C | 92,5 % |
| 25 | H | Cl | H | H | Cl | H | 205°C | 94,5 % |
| 26 | H | Cl | Cl | H | H | Cl | 204°C | 91,9 % |
| 27 | $CH_3$ | H | Cl | $CH_3$ | H | Cl | 190°C | 93,2 % |
| 28 | Cl | H | Cl | Cl | H | Cl | 164°C | 95,6 % |
| 29 | H | Br | H | H | Br | H | 206°C | 94,6 % |
| 30 | H | $NO_2$ | H | H | $NO_2$ | H | 242°C | 91,8 % |

**Patentanspruch**

Verfahren zur Herstellung von 2-Aminophenyl-thioethern der allgemeinen Formel (1)

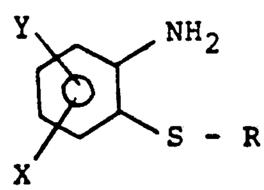

(1)

in welcher R eine Alkyl$_{C_1-C_6}$gruppe, die durch Hydroxyl-, Alkoxy$_{C_1-C_4}$, Carboxyl-, —COO-Alkyl$_{C_1-C_4}$, Alkenyl$_{C_2-C_6}$ oder Phenylgruppen oder den Rest

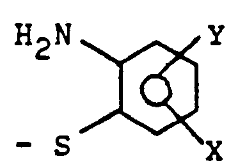

substituiert sein kann, und X und Y je ein Wasserstoff- oder Halogenatom, oder eine Alkyl$_{C_1-C_6}$, Alkoxy$_{C_1-C_6}$ oder Nitrogruppe darstellen, dadurch gekennzeichnet, daß man die in bekannter Weise durch Umsetzung von 2-Aminobenzthiazolen der allgemeinen Formel (3)

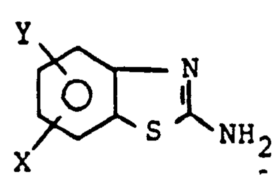

(3)

in welcher X und Y die vorstehend genannten Bedeutungen haben, mit Alkalimetall- oder Erdalkalimetallhydroxiden in einem alkalibeständigen wasserfreien oder praktisch wasserfreien Lösungsmittel erhältichen Salze der allgemeinen Formel (4)

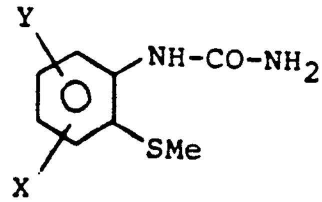

(4)

in welcher X und Y die vorstehend genannten Bedeutungen haben und Me ein Alkalimetallatom oder die äquivalente Menge eines Erdalkalimetallatoms bedeutet, in isolierter Form oder vorteilhaft in der im Ringöffnungsgemisch vorliegenden Suspension mit einem Alkalierungsmittel der allgemeinen Formel (2)

$$R—Z \qquad (2)$$

in welcher R die vorstehend genannte Bedeutung hat und Z ein Halogenatom darstellt, oder der allgemeinen formel (2a)

$$(R'O)_2SO_2 \qquad (2a)$$

in welcher R' eine Alkyl$_{\overline{C_1-C_4}}$ gruppe bedeutet, oder der allgemeinen Formel (2b)

$$Z\text{-alkylen}_{\overline{C_2-C_6}}\,Z \qquad (2b)$$

in welcher Z die vorstehend genannte Bedeutung hat, oder mit einem Alkylen$_{\overline{C_2-C_3}}$oxid bei Temperaturen von 0° bis 100°C umsetzt.

**Revendication**

Procédé pour préparer des thio-éthers amino-2 phényliques répondant à la formule générale (1):

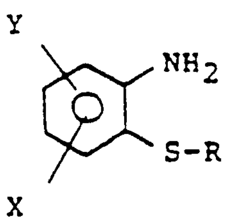

(1)

dans laquelle

R représente un alkyle en $C_1$—$C_6$ éventuellement porteur d'un hydroxy, d'un alcoxy en $C_1$—$C_4$, d'un carboxy, d'un alcoxycarbonyle à alkyle en $C_1$—$C_4$, d'un alcényle en $C_2$—$C_6$ ou d'un phényle, ou représente un radical

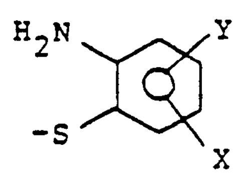

et X et Y représentent chacun un atome d'hydrogène, un atome d'halogène, un alkyle en $C_1$—$C_6$, un alcoxy en $C_1$—$C_6$ ou un radical nitro, procédé caractérisé en ce qu'on fait réagir, à des températures de 0 à 100°C, les sels répondant à la formule générale (4):

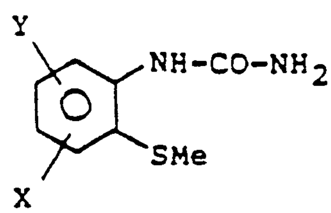

(4)

dans laquelle X et Y ont les significations indiquées ci-dessus, et Me représente un atome de métal alcalin ou la quantité équivalente d'un atome de métal alcalino-terreux — sels qui peuvent être obtenus de manière connue par réaction d'amino-2 benzothiazoles répondant à la formule générale (3):

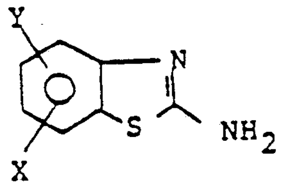

(3)

dans laquelle X et Y ont la signification indiquée ci-dessus, avec des hydroxydes de métaux alcalins ou de métaux alcalino-terreux, dans un solvant stable aux alcalis, anhydre ou pratiquement anhydre — à l'état isolé ou, mieux, en suspension dans le mélange provenant de la réaction d'ouverture du cycle, avec un agent d'alkylation répondant à la formule générale 2:

R—Z (2)

dans laquelle R a la signification indiquée ci-dessus et Z représente un atome d'halogène, ou répondant à la formule

$(R'O)_2SO_2$ (2a)

dans laquelle R' représente un alkyle en $C_1$—$C_4$, ou répondant à la formule générale (2b):

Z-alkylene$_{\overline{C_2-C_6}}$ Z (2b)

dans laquelle Z a la signification indiquée ci-dessus, ou encore avec un oxyde d'alkylène contenant deux ou trois atomes de carbone.

**Claim**

A process for the preparation of 2-aminophenyl thioethers of the formula (1)

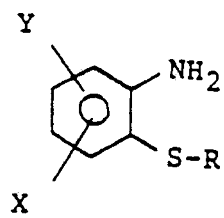

(1)

in which R represents an alkyl$_{\overline{C_1-C_6}}$ group which can be substituted by hydroxyl, alkoxy$_{\overline{C_2-C_4}}$, carboxyl, —COO-alkyl$_{\overline{C_1-C_4}}$, alkenyl$_{\overline{C_2-C_6}}$ or phenyl groups or by the radical

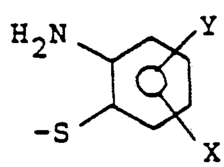

and X and Y are each a hydrogen or halogen atom or an alkyl$_{\overline{C_1-C_6}}$, alkoxy$_{\overline{C_1-C_6}}$ or nitro group, wherein the salts of the formula (4)

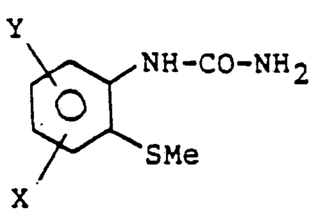

(4)

in which X and Y have the abovementioned meanings and Me denotes an alkali metal atom or the equivalent amount of an alkaline earth metal atom, which are obtainable in a known manner by reacting 2-aminobenzothiazoles of the formula (3)

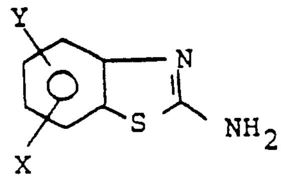

(3)

in which X and Y have the abovementioned meanings, with alkali metal or alkaline earth metal hydroxides in an alkali-stable anhydrous or virtually anhydrous solvent, are reacted, in the isolated form or, advantageously, in suspension in the mixture obtained in the ring-opening step, with an alkylating agent of the formula (2)

$$R—Z \quad (2)$$

in which R has the abovementioned meaning and Z represents a halogen atom, or of the formula (2a)

$$(R'O)_2SO_2 \quad (2a)$$

in which R' denotes an $alkyl_{\overline{C_2-C_6}}$ group, or of the formula (2b)

$$Z\text{-}alkylene_{\overline{C_2-C_6}}\,Z \quad (2b)$$

in which Z has the abovementioned meaning, or with an alkylene $C_2$ or $C_3$-oxide at temperatures of 0 to 100°C.